# EUROPEAN PATENT APPLICATION

(11) **EP 1 127 942 A1**
(43) Date of publication of application: **29.08.2001**
(21) Application number: 01830116.8
(22) Date of filing: 21.02.2001
(51) Int. Cl.: C12N 15/11, C12N 5/08, C07K 14/47, C12Q 1/02

(54) **Method of immortalization of human keratinocytes by down-regulation of 14-3-3 sigma expression**

(30) Priority: 25.02.2000 IT RM000095
(71) Applicant: Provincia Italiana Della Congregazio ne Dei Figli Dell'Immacolata Concene-Instituto Dermopatico Dell'Immacolata, 00167 Roma (IT)
(72) Inventor: De Luca, Michele, 00040 Ardea (IT); Dellambra, Elena, 00040 Ardea (IT)
(74) Representative: Sarpi, Maurizio

(57) **Abstract**

Immortalised cell lines from primary human keratinocytes cell lines obtained thanks to down regulation of 14-3-3-sigma coding gene. Said immortalised cell lines are extremely useful in *in vivo* and *in vitro* studies on cancer development. Infact, immortalization is absolutely required for somatic cells transformation. It is also an object of the present invention the immortalised cell lines obtained thereof.

## Description

### SUMMARY OF THE INVENTION

The present invention relates to a method that leads to immortalization of primary human keratinocytes cell cultures.

According to this invention, cell culture immortalization is obtained down regulating the expression of the gene coding for 14-3-3-sigma.

Immortalization allows cultured cell lines, obtained from a primary cell line, to live indefinitely. In this invention, unlike other well-known procedures, immortalization is obtained without the need of artificially introduced exogenous oncoviruses or oncogenes. Immortalized cells, related to this invention, are much more similar to wild type cells than those obtained with others well-known methods. Said immortalized cells, according to this invention, are extremely useful for further studies about treatments and diagnostic methods on cell changes related to skin cancers, since said cancers types are now the most prevalent malignancy in light skinned population world wide.

### BACKGROUND OF THE INVENTION

It's well known that telomere shortening, which occurs during cell division, and the activation of tumor suppressor gene, like P53, p21/cip1/WAF1 e p16/INK4a are considered essential steps in determining the exit of senescent cells from the cell cycle. The link between the control of proliferative capacity of somatics cells and the control of the cell cycle (and obviously with the absence of telomerase activity and the activation of tumor suppressor genes) is surely a natural way to prevent and control the onset and development of cancers.

The opportunity to block senescence of a primary coltured cell line and the immortalization of a primary cell line requires multiple genetic changes, like the inactivation of tumor suppressor genes and resumption of telomerase activity (Reddel,R., 1998, A reassessment of the telomere hypothesis of senescence, BioEssays, 20, 977-984; Greider,C.W., 1999, Telomerase activation: one step on the road to cancer?, Trends in Genet.,15,109-112; Wynford-Thomas, D., 1999, Cellular senescence and cancer, J. Pathol.,187,100-111). Maintenance of telomere length is necessary, and in certain cases sufficient, to immortalise cells. For instance, constitutive expression of the catalytic component of telomerase (hTERT) apparently leads to one-step immortalization in human fibroblasts (Bodnar et al., 1998, Extension of life span by introduction of telomerase into normal humans cells, Science, 279, 349-352). Whereas immortalization of human keratinocytes requires both exogenous hTERT and inactivation of the p16/INK4a /pRb pathway (Kiyono et al., 1998, Nature, 396, 84-88). Accordingly, human keratinocyte immortalization requires the co-ordinate action of HPV E6 and E7 oncogenes, the former being responsible for telomerase activation and for p53 degradation, the latter being responsible for telomerase activation (Reddel, 1998,op. cit.; Greider,1999, op.cit.).

### DESCRIPTION

The expression of 14-3-3-sigma (also called stratifin or HME1) is highly specific for human stratified epithelia. We reported that the down-regulation of 14-3-3-sigma leads to immortalization of primary human epidermal keratinocytes, without the need of exogenous oncogenes and/or oncoviruses. Down regulation of sigma expression, obtained using the antisense mRNA, allows keratinocytes to escape senescence. The immortalization is accompanied by resumption of telomerase activity and by strong down regulation or inactivation of the p16/INK4a tumor suppressor gene.

Further advantages and characteristic of the present invention will be more readily apparent from the followig description, with reference to the enclosed drawings in which:
figg. 1A and 1B, 2A and 2B, 3A and 3B show proliferation capabilities of three samples of primary coltured keratinocytes cells transferred with antisense-14-3-3 sigma-cDNA (open circles) or with empty vectors (black squares);
figg. 4A and 4B compare telomerase activity and p16/INK4a expression in keratinocytes cultured cells transferred with antisense-sigma-DNA, empty vectors or untrasfected cells.

The immortalization method, which is the object the present invention, is developed from the results showing that down regulation of 14-3-3-sigma induces keratinocytes immortalization. The immortalization is accompanied by resumption of telomerase activity and by strong down-regulation of the p16/INK4a tumor suppressor gene.

While most of 14-3-3 proteins are ubiquitously expressed, sigma is highly specific for human stratified epithelia. (Leffers et al., 1993, J.Mol.Biol., 231, 982-988).

Expression of sigma was previously investigated on skin biopsies obtained from healthy donors. *In situ* hybridization revealed that sigma mRNA was barely detectable in epidermal basal layer, but abundantly expressed in suprabasal layers. Sigma was undetectable in the stratum corneum.

Cultured keratinocytes generate cohesive sheets of stratified squamous epithelium that differs from the corresponding natural epidermis mainly because it is formed by more flat cells, it has fewer suprabasal layers and it lacks the stratum corneum. Like in natural epidermis, very low amounts of sigma expression were detected in the basal layer of cultured epidermis, otherwise maximal expression of sigma was observed immediately above the basal layer. Thus Sigma is expressed, *in vivo* and *in vitro* in areas of epidermis where cell proliferation has ceased and where the major markers of terminal differentiation are synthesised (Green,H., 1980, Harvey Lect., 74,101-139; Fuchs, E., 1990, Epidermal differentiation: the bare essentials, J Cell Biol, 111, 2807-2814; Roop,D., 1995, Defects in the barrier, Science, 267, 474-475).

In cultured epidermis, it is quite common to observe basal cells in transition from basal to suprabasal position. These cells usually express high amounts of sigma, suggesting that sigma might play a role in regulating both the exit from the cell cycle and the progression through terminal differentiation.

Cellular size is a major determinant of keratinocytes clonogenic ability and terminal differentiation. Clonogenic cells capable of multiplication are the smallest cells in the epithelium. Upon commitment to terminal differentiation, keratinocytes increase their size and express involucrin, which is considered as an early-intermediate differentiation marker. The epidermal basal layer is involucrin-negative and suprebasal layers are involucrin-positive. To examine the relation between the expression of sigma, the potential for multiplication and the expression of involucrin, confluent sheets of primary keratinocytes were trypsinized and cells were separated according to their size by centrifugal elutriation. Six fractions were collected and cell diameter increased progressively from approximately 10 µm to 30-50 µm. To monitor proliferative potential, cells of each fraction were tested for their ability to form colonies. Colony forming cells were concentrated almost entirely in the first two fractions (10-20 µm), whereas cells of the three fractions (30-50 µm) lost virtually all colonies forming capability. Colonies of each fraction were then analysed for the expression of both sigma and involucrin. Sigma was barely detectable in the first fraction and its expression increased slightly in the second fraction. A sharp increase of sigma expression was observed in the third and fourth fractions (20-30 µm), to coincide with the abrupt drop of clonogenic ability. Accordingly, involucrin was absent in the first two fractions, its appearance coincided with the sharp rise of sigma observed in the third fraction, and its expression remained high afterwards.

The evidence that 14-3-3-sigma inhibition leads to immortalization of primary cultured keratinocytes cells is based on the previous results.

It is demonstrated that 14-3-3-sigma inhibition allows primary cultured keratinocytes to escape from senescence. Its is due to resumption of telomerase activity and by strong down-regulation of the p16/INK4a tumor suppressor gene.

Immortalization procedures, used in this invention, are completely different from those using oncogenes and/or oncoviruses. Down-regulation of 14-1-3-3-sigma expression leads to immortalization of primary human epidermal keratinocytes, without introducing exogenous oncogenes (as E6 and E7 human papilloma virus genes) and\or transfetting with oncoviruses. Obtained cell lines are tested *in vitro* to evaluate the onset of tumoral phenotypes.

Down-regulation of 14-1-3-3-sigma expression leads to immortalization of primary human epidermal keratinocytes without having a tumoral phenotype like cells produced using oncoviruses and/or oncoviruses.

### EXAMPLES OF CELL LINES IMMORTALIZATION PROCEDURES

Three different samples of primary cultured keratinocytes were generated by transfection with a difective retroviral vector containing the full length human sigma cDNA in antisense orientation, as previously described in specific transfection protocols for transiently amplification of steam cells.

Therefore, the obtained down regulation of sigma expression recapitulates the combined action of Human papilloma virus E6 and E7 oncogenes, and place 14-3-3sigma upstream of two key events in human keratinocyte immortalization.

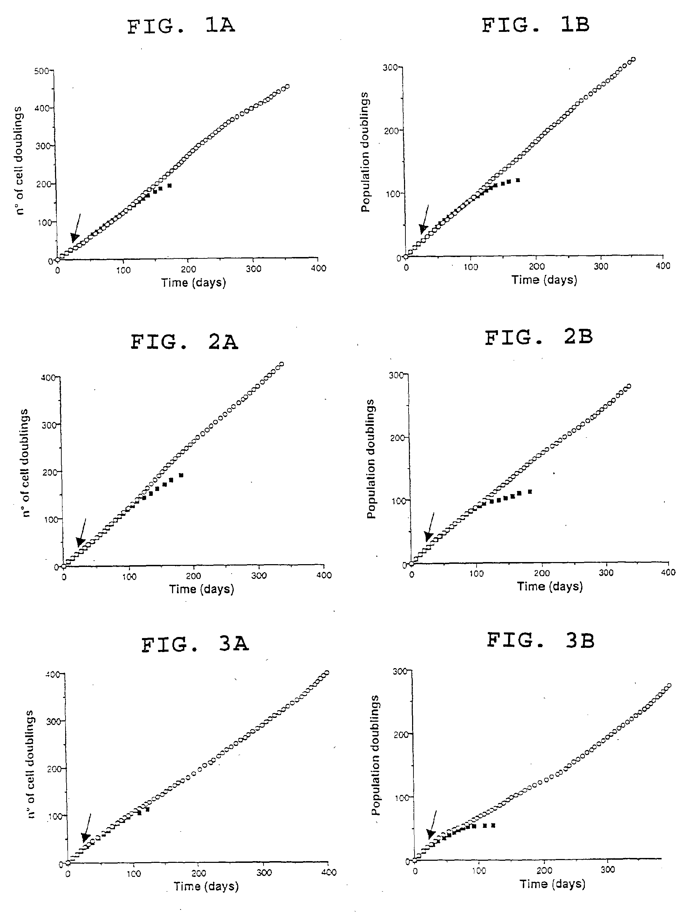

Figg. 1A-B,2A-B and 3A-B, show the proliferative capability of three different samples of primary cultured keratinocytes transfected with antisense-sigma-DNA (open circles) or empty vectors (black squares). As shown, cells transduced with empty vectors (black squares), after a certain number of cell passages underwent replicative senescence. In contrast, antisense-sigma-transduced Keratinocytes continued to divide indefinitely.

As shown in fig. 4A, telomerase activity was detected using the PCR-based, telomeric repeat amplification protocol (TRAP) assay in both untransduced (C-1), vector-trasduced (V-1) and antisense-sigma-transduced (AS-1) keratinocytes (lanes AS9 lines referred to different cell divisions, indicates by numbers). Telomerase activity was clearly detectable in untransduced cells (C-1), but undetectable in control cells, transduced with empty vectors, close to replicative senescence (V-17). In contrast, antisense-sigma-transduced keratinocytes fully resumed telomerase activity at later cells passages after bypass of senescence (AS-19,AS-49). T.L. indicates telomeres length (Kb means Kilobases).

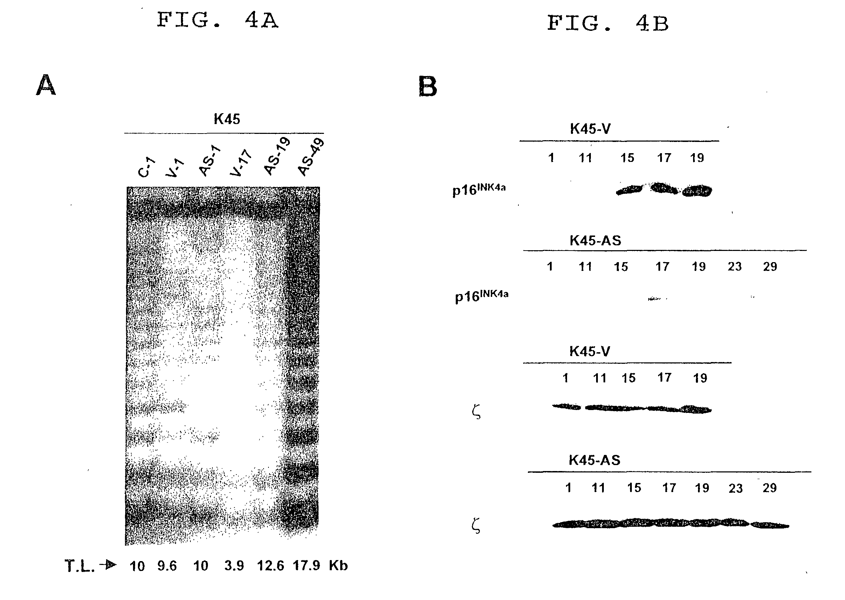

Fig. 4B shows the results of Western analysis on cell extract prepared from control cells close to senescence (V-lanes) and from antisense-sigma-transduced cells (AS-lanes) at different cell passages (indicated by numbers) after infection. p16/INK4a was not detectable in control cells at early generations and in antisense-sigma-transduced cells, but its maximal expression was evidenced in control cells approaching senescence (V15-19). Identical results were obtained in all transduced cell strains.

In summary, keratinocytes transduced with antisense sigma did not simply extend their life span, but have been immortalized. Down regulation of sigma expression leads to down regulation of p16^{INK4a} and the resumption of telomerase activity, which is invariably associated to cellular immortalization.

## Claims

1. Method of immortalization of primary cell cultures of human keratinocytes, wherein said immortalization is obtained by down regulation and/or suppression of the expression of 14-3-3-sigma coding gene.

2. Method of immortalization of primary cell cultures of human keratinocytes according to claim 1, wherein down regulation of sigma expression is obtained by blocking sigma-coding-mRNA with a RNA in antisense orientation.

3. Method of immortalization of primary cell cultures of human keratinocytes according to claim 1 wherein said method is obtained without the need of artificially introduced exogenous oncoviruses or oncogenes.

4. Method of immortalization of primary cell cultures of human keratinocytes according to claim 1 wherein the immortalization is accompanied by resumption of telomerase activity and by down-regulation or inactivation of the p16/INK4a tumor suppressor gene.

5. Method of immortalization of primary cell cultures of human keratinocytes according to claim 1 wherein down regulation of sigma expression is obtained by transfection with a defective retroviral vector containing the full length human sigma cDNA in antisense orientation, as described in specific transfection protocols for steam cells and transiently amplificated steam cells.

6. Cultured cell lines obtained from immortalized primary cell cultures of human keratinocytes by down regulating or inactivating 14-3-3-sigma expression.

7. Cultured cell lines according to claim 6 wherein said cultured cell lines are capable to live indefinitely without the need of artificially introduced exogenous oncoviruses or oncogenes and do not show any tumoral phenotype like cell lines produced using oncoviruses.

8. Immortalized cultured cell lines according to claim 6 wherein said immortalized cultured cell lines are suitable in studies about treatments and diagnostic methods for skin cancers.

9. Immortalized cultured cell lines according to claim 6 wherein said immortalized cultured cell lines are suitable in studies about treatments and diagnostic methods for skin cancers.
